# EUROPEAN PATENT SPECIFICATION

(11) **EP 1 617 946 B1**
(45) Date of publication and mention of the grant of the patent: **08.09.2010**
(21) Application number: 03816516.3
(22) Date of filing: 31.03.2003
(51) Int. Cl.: B01J 21/06, B01J 21/16, B01J 29/04, B01J 27/18, C07D 213/00

(54) **TI-PILLARED CLAY BASED VANADIA CATALYST AND PROCESS FOR PREPARATION**
TI-GETRÄGERTER VANADIUMKATALYSATOR AUF TONERDEBASIS UND HERSTELLUNGSVERFAHREN
CATALYSEUR DE VANADIUM A BASE D'ARGILE A PILIER DE TITANE (TI) ET PROCEDE DE PREPARATION DE CELUI-CI

(43) Date of publication of application: 25.01.2006
(73) Proprietor: Council of Scientific and Industrial Research, New Delhi 110 001 (IN)
(72) Inventor: ROY, Shyam K., Central Fuel Research Institute, Dhanbad, Jharkhand (IN); ROY, Subhash C., Central Fuel Research Institute, Dhanbad, Jharkhand (IN); DUTTA, Pasupati, Central Fuel Research Institute, Dhanbad, Jharkhand (IN); NANDI, Laxmi N., Central Fuel Research Institute, Dhanbad, Jharkhand (IN); YADAV, Satya, N., Central Fuel Research Institute, Dhanbad, Jharkhand (IN)
(74) Representative: Jennings, Nigel Robin
(86) International application number: PCT/IN2003/000101
(87) International publication number: WO 2004/087310

(56) References cited:
- US-A- 2 510 605
- US-A- 2 861 999
- US-A- 3 970 659
- US-A- 3 981 879
- US-B1- 6 475 944

## Description

### Field of the invention

The invention relates to a pillared clay based vanadia catalyst. The present invention also relates to a process for the preparation of vanadia based catalyst supported on titanium-pillared clay useful for producing heteroaromatic nitriles. The present invention also relates to a process for the preparation of pillared clay based vanadia catalyst useful for production ofnicotinonitrile (3-cyanopyridine) by ammoxidation of 3-picoline and to the use thereof for the production of 3-cyanopyridine.

### Background of the invention

The present invention finds its usage in preparing valuable intermediates for the preparation of corresponding amides, which are used in pharmaceuticals. 3-cyano-pyridine is used in the preparation of niacin, used for the treatment of pellagra. Many drugs based on niacin have curative effects such as lowering the limit of cholesterol and free fatty acids in blood, stimulation of the respiratory apparatus, antispasmodic and anti-rheumatic action e.g. the antispasmodic lyspamin and coramine or Nikethamide and Bilamid used in treating infections of gallbladder or bile duct.

Reference is made to US Patent 5,614,453 which relates to acid catalyzed chemical conversion processes, such as hydrocarbon conversion processes, and to the catalysts and catalyst supports used in such processes. The invention is particularly concerned with catalyst supports containing a combination of zeolite Beta and a pillared clay, catalysts comprising such supports and the use of such catalysts in hydrocarbon conversion processes, particularly hydrocracking. There is no mention about conversion of 3-picoline. Reference is also made to US Patents 2,510,605, 2,839,535 and 2,861,999 wherein the catalysts based on vanadium, molybdenum and phosphorous on activated alumina has been described. However, the yields of nicotinonitrile and isonicotinonitrile were in the range of 60 to 70 %. Reference is also made to US Patent 3981879 wherein catalysts based on vanadium, molybdenum, phosphorous oxides and tin oxide over pumice have been described which gives 90% selectivity at 76% conversion. Reference is also made to Japanese Patent 19706 wherein a catalyst has been disclosed, comprising of antimony oxide, vanadium oxide containing a metal from group (iron, copper, titanium, cobalt, manganese and nickel with high selectivity but it deactivates due to reduction by ammonia.

Pillared clays are typically prepared by reacting a smectite clay such as montmorillonite, saponite, hectorite, and beidellite with a pillaring agent or propping agent to form a flocculated reaction product which is subsequently dried to convert the pillaring agent into inorganic metal oxide clusters which form the pillars which prop apart the layers of the clay. The X-ray diffraction pattern of a pillared clay normally contains a distinct first order reflection which is indicative of a well ordered, long range, face-to-face orientation of the clay platelets.

Prior art search for a process for preparation of titanium based pillared clay catalyst for conversion of 3-picoline to 3-cyanopyridine has been made based on literature survey and patent databases, which did not yield any relevant references.

### Objects of the invention

The main object of the present invention is to provide a process for the preparation of a catalyst supported on titanium-pillared clay useful for producing heteroaromatic nitriles.

Another object of the present invention is to provide a process for the preparation of pillared clay based vanadia catalyst useful for production of nicotinonitrile (3-cyanopyridine) by ammoxidation of 3-picoline.

Yet another object of the present invention is to convert 3-picoline into nicotinonitrile in a single step reaction between 3-picoline, ammonia and air.

Still another object of the present invention is to provide a process for preparing 3-cyano pyridine in high yield ( >90%).

### Summary of the invention

Accordingly the present invention provides a pillared clay based vanadia catalyst useful for the conversion of 3-picoline to 3-cyanopyridine, said catalyst comprising vanadium, phosphorous and molybdenum on a Ti-pillared clay support.

In one embodiment of the invention, the clay is pillared with polyoxymetal cations of titanium and has titania clusters as the pillars between the layers.

In another embodiment of the invention, the clay is selected from a single smectite clay and mixed layer smectite clays.

In another embodiment of the invention, the single smectite clay comprises montmorillonite clay.

In another embodiment of the invention, the mixed layered smectite clay is selected from rectorite and paragonite.

In another embodiment, the pillared clay contains a homogeneous distribution of pillars in the interlayered spaces thereof forming an array of rectangular openings or pores of a size in the range of 7 to 20 Angstroms high and between 8 and 20 Angstroms wide, enabling the pillared clay to perform like a two-dimensional crystalline molecular sieve.

In still another embodiment of the invention the ratio of the compounds of vanadium, molybdenum, phosphorous over titanium pillared clay in the catalyst is in a ratio ranging between 1.0: 2.5: 0.5: 20 - 1.0: 30: 1.5: 50.

In a further embodiment of the invention, the pillared clay contains one or more rare earth elements selected from cerium, lanthanum and a mixture thereof.

The present invention also relates to a process for the preparation of a pillared clay based vandia catalyst comprising first preparing vanadyl oxalate by heating a vanadium rich compound on a water bath followed by addition of oxalic acid till the colour of the mass changes to violet blue indicating the formation of vanadyl oxalate, said vanadyl oxalate being in dilute solution, adding a phosphorous source to the dilute solution of vanadyl oxalate heated over water bath, followed by addition of an aqueous solution of molybdenum source and mixing while adding a Titanium source under stirring, evaporating the solution over water bath till the mass is reduced to about half, further heating the mass till a powder catalyst is obtained.

In an embodiment of the present invention vanadium rich compound is selected from the group consisting of ammonium metavanadate, vanadyl sulphate and oxides of vanadium.

In another embodiment, the oxide of vanadium is vanadium pentoxide.

In another embodiment of the invention the phosphorous source is selected from the group consisting of ortho-phosphoric acid, pyro-phosphoric acid and meta-phosphoric acid.

In yet another embodiment the molybdenum source is ammonium molybdate.

In another embodiment of the invention the titanium source is titanium-pillared clay.

In one embodiment of the invention, the clay is pillared with polyoxymetal cations of titanium and has titania clusters as the pillars between the layers.

In another embodiment of the invention, the clay is selected from a single smectite clay and mixed layer smectite clays.

In another embodiment of the invention, the single smectite clay comprises montmorillonite clay.

In another embodiment of the invention, the mixed layered smectite clay is selected from rectorite and paragonite.

In a further embodiment of the invention, the pillared clay contains one or more rare earth elements selected from cerium, lanthanum and a mixture thereof

In another embodiment of the invention the ratio of the compounds of vanadium, molybdenum, phosphorous over titanium pillared clay in the catalyst is in a ratio ranging between 1.0: 2.5: 0.5: 20 - 1.0: 30: 1.5: 50.

In another embodiment of the invention the reaction is carried out at a space velocitie in the range of 1500 to 5500 h ⁻¹ and after dilution of the catalyst with an inert medium to a range in the extent of 0.5 to 4 by volume with respect to the volume of catalyst.

In another embodiment of the invention, the reduced mass is heated at a temperature of about 110°C in an air oven for 15 hours and then further heated in a muffle furnace at a temperature of about 300°C for 3 hours and subsequently at a temperature of about 425°C for 15 hours.

In yet another embodiment of the invention, the catalyst powder obtained is pelletized and sized to -6 to +14 mesh size.

In another embodiment of the invention, the vanadium rich compound comprises vanadium pentoxide, said process comprising heating vanadium pentoxide with water in the range of 1:3 to 1:5 (w/v) followed by addition of oxalic acid in the range of 2.5 to 3.0 parts by weight of vanadium pentoxide till the colour of the mass changes to violet blue; adding phosphorous source to the dilute solution of vanadyl oxalate in the range of 2 to 4 times by volume of vanadyl oxalate over heating bath at a temperature in the range of 70 to 80°C; preparing aqueous solution of molybdenum source in the range of 1:4 to 1:6 (w/v) and heating over a heating bath at a temperature in the range of 70 to 80°C; mixing both the solutions in heating condition over heating bath with appropriate amount of titanium-pillared clay under stirring; evaporating the mixture over a heating bath till the mass reduces to half; heating the mass in an air oven at a temperature in the range of 100 to 120°C for a time period in the range of 10 to 16 hours and in a muffle furnace at a temperature in the range of 250 to 350°C for a time period in the range of 16 to 20 hours and pelletizing the same size range of -6 to +14 BSS.

The present process also provides a process for the conversion of 3-picoline into 3-cyanopyridine in a single step, said process comprising reacting 3-picoline, ammonia and air in the presence of a Ti-pillared clay based vanadia catalyst to obtain 3-cyanopyridine, without requiring addition of oxidizing agents.

In one embodiment of the invention, the clay is pillared with polyoxymetal cations of titanium and has titania clusters as the pillars between the layers.

In another embodiment of the invention, the clay is selected from a single smectite clay and mixed layer smectite clays.

In another embodiment, the single smectite clay comprises montmorillonite clay.

In another embodiment of the invention, the mixed layered smectite clay is selected from rectorite and paragonite.

In a further embodiment of the invention, the pillared clay contains one or more rare earth elements selected from cerium, lanthanum and a mixture thereof.

In still another embodiment of the invention the ratio of the compounds of vanadium, molybdenum, phosphorous over titanium pillared clay in the catalyst is in a ratio ranging between 1.0: 2.5: 0.5: 20 - 1.0: 30: 1.5: 50.

In another embodiment of the invention, the pillared clay contains a homogeneous distribution of pillars in the interlayered spaces thereof forming an array of rectangular openings or pores of a size in the range of 7 to 20 Angstroms high and between 8 and 20 Angstroms wide, enabling the pillared clay to perform like a two-dimensional crystalline molecular sieve.

### Detailed description of the invention

The novelty of the present invention lies in providing a catalyst for conversion of 3-picoline to 3-cyanopyridine by reaction of ammonia and air with 3-picoline in a single step, yielding a high purity product, eliminating expensive oxidising agents and enabling the operation of the process on a continuous basis and the inventive step lies in providing non-obvious steps of the process in an environmental friendly manner in comparison to prior art processes.

A preferred pillared clay for use in the catalyst of the invention has been pillared with polyoxymetal cations of titanium and therefore has titania clusters as the pillars between the layers. Besides a single smectite clay such as montmorillonite, mixed layered smectites such as rectorite and paragonite can also be pillared and used in the catalyst of the invention. Examples of pillared clays which possess stability at high temperatures are ones in which the pillars contain one or more rare earth elements such as cerium and/or lanthanum. Such clays are disclosed in U.S. Pat. Nos. 4,753,909 and 4,952,544 and in PCT International Application WO 88/06614, the disclosures of which patents and application are hereby incorporated by reference in their entireties.

It will be understood, however, that pillared clays in which the pillars are substantially free of rare earth elements can be used as a component of the catalyst. Pillared clays and their preparation are described generally in an article entitled "Intercalated Clay Catalysts," Science, Vol. 220, No. 4595, pp. 365-371 (Apr. 22, 1983), the disclosure of which is hereby incorporated by reference in its entirety.

The following examples are given by way of illustration of the present invention and should not be construed to limit the scope of the present invention.

### Example 1

Preparation of Ti-Pillared clay: TiO₂- PILC (Titanium pillared clay) was synthesized by pillaring montmorilonite /bentonite clay (sodium ion exchanged) by a solution of partially hydrolysed Ti-polycations. Ti-polycation was prepared by adding the concentrated solution of TiCl₄ into 2 moles of HCl( Hydrochloric Acid). The mixture was then diluted by slowly adding water with constant stirring so that final concentration of Titanium in the solution was 0. 82 mole and final concentration of HCl was 0.6 mole.

8 g Sodium exchanged clay was dispersed in 2 litre distilled water. The slurry was stirred for 8 h by mechanical stirrer. The Titanium polycation pillaring agent was added to the slurry slowly with vigorous stirring at 90°C. The solution contained 10 m mole titanium/g clay. The resulting solution was further stirred for 4 h. The solution was kept 18 h at room temperature. The mass was filtered under suction and thoroughly washed with distilled water to make chloride ion free. The product is oven dried at 110±10°C for 6 h. Finally it was calcined at 350°C for 12 h in a muffle furnace.

Preparation of Vanadyl oxalate: 45 g vanadium pentoxide in water(1:5) was heated over steam bath and 122 g oxalic acid crystals slowly added pinch wise with stirring till the colour of the slurry changes from yellow to violet blue. It was further heated over steam bath for 15 minutes. Thus the solution of vanadyl oxalate was prepared.

Preparation of the catalyst and its use in ammoxidation of 3-picoline: 6.6 ml vanadyl oxalate solution containing 3.1 g vanadyl oxalate (equivalent to 1.8 g vanadium pentoxide) as prepared above was taken in a porcelain basin. It was diluted with 40 ml of distilled water, mixed thoroughly with stirring and heated at 70-80 °C. To this solution 0.05 g H₃PO₄ (85%) was added and mixed thoroughly. Another solution was made using 0.4 g molybdenum trioxide and 5 ml of (1:1) ammonium hydroxide and heated at 70-80°C. Both the solutions were mixed with stirring at the same temperature. 7.75 g Ti-PILC was added to the mixed solution with constant stirring. It was stirred for another 15 minutes. The mixed mass was heated on a steam bath. This was then heated in an air oven at 110°C for 15 h, then in a muffle furnace for 3 h at 300°C and further at 425°C for 15 h. The powder catalyst was pelletized and used in the form of -6 to +14 BS mesh.

The reaction was carried out using a down flow, fixed bed, pyrex glass reactor of 20 mm i.d.. The reactor is packed with 10 ml (8 g ) Ti-PILC based catalyst. The reaction mixture was fed from top of the reactor. 3-Picoline and water in the ratio 1:3 (by vol) was fed at the rate 4.35 ml/h using a syringe pump (Sage instrument). Ammonia gas(2.1 1/h) from gas cylinder and air (4.5 1/h) through calibrated flow meter were fed in to the reactor. The reaction was carried out at temperature of 425 ± 10°C and contact time 1.4 s (space velocity 2571 cc/cc cat/h). The product was cooled using ice cooled water and collected at the bottom followed by two number of ice traps. The product was analysed by gas chromatograph using 2 m length carbowax 20 M column at 160°C temperature and Thermal Conductivity Detector. The yield of the product (3-cyanopyridine) was 80.6% where as the conversion of 3-picoline was 87.6 %

### Example 2

Preparation of Ti-Pillared clay: TiO₂- PILC ( Titanium pillared clay) was synthesised by pillaring montmorilonite /bentonite clay (sodium ion exchanged) by a solution of partially hydrolysed Ti-polycations. Ti-polycation was prepared by adding the concentrated solution of TiCl₄ into 2 moles of HCl( Hydrochloric Acid). The mixture was then diluted by slowly adding water with constant stirring so that final concentration of Titanium in the solution was 0.82 mole and final concentration of HCl was 0.6 mole.

8 g Sodium exchanged clay was dispersed in 2 litre distilled water. The slurry was stirred for 8 h by mechanical stirrer. The Titanium polycation pillaring agent was added to the slurry slowly with vigorous stirring at 90°C. The solution contained 10 m mole titanium/g clay. The resulting solution was further stirred for 4 h. The solution was kept 18 h at room temperature. The mass was filtered under suction and thoroughly washed with distilled water to make Cl⁻ ion free. The product is oven dried at 110±10°C for 6 h. Finally it was calcined at 350°C for 12 h in a muffle furnace.

Preparation of Vanadyl oxalate: 45 g vanadium pentoxide in water(1:5) was heated over steam bath and 122 g oxalic acid crystals slowly added pinch wise with stirring till the colour of the slurry changes from yellow to violet blue. It was further heated over steam bath for 15 minutes. Thus the solution ofvanadyl oxalate was prepared.

8.9 ml vanadyl oxalate solution containing 3.1 g vanadyl oxalate (equivalent to 1.8 g. vanadium pentoxide) was taken in a porcelain basin. It was diluted with 40 ml of distilled water, mixed thoroughly with stirring and heated at 70-80°C. To this solution 0.1 g H₃PO₄ (85%) was added and mixed. Another solution was made using 0.4 g molybdenum trioxide and 5 ml of (1:1) ammonium hydroxide and heated to 70-80°C. This solution was added to the first solution with stirring keeping the temperature at about 80°C. 7.75 g Ti-PILC was added to the mixed solution with constant stirring. It was stirred for another 15 minutes. The mixed mass was evaporated on a steam bath. This was then oven dried at 110-120°C for 15 h, then calcined in a muffle furnace for 3 h at 300°C and further at 425°C for 15 h. The powder catalyst was pelletised and used in the form of -6 to +14 BS mesh.

The reaction was carried out using a down flow fixed bed pyrex glass reactor of 20 mm i.d. The reactor was packed with 5 ml (4.38 g) Ti-PILC based catalyst. The reaction mixture was fed from the top of the reactor 3-picoline and water in the ratio of 1:3 by vol. was fed at the rate of 4.3 ml/h using a syringe pump (Sage instrument). Ammonia gas (2.10 1/h) and air (4.5 1/h) from cylinder through calibrated flow meter were fed into the reactor. The reaction was carried out at temperature 440±10 °C and contact time 0.66 s (space velocity 5438-cc/cc cat/h). The yield obtained was 91% conversion 98.0%.

### Example 3

Preparation of Ti-Pillared clay: TiO₂- PILC ( Titanium pillared clay) was synthesised by pillaring montmorillonite /bentonite clay (sodium ion exchanged) by a solution of partially hydrolysed Ti-polycations. Ti-polycation was prepared by adding the concentrated solution of TiCl₄ into 2 moles of HCl (Hydrochloric Acid). The mixture was then diluted by slowly adding water with constant stirring so that final concentration of Titanium in the solution was 0.82 mole and final concentration of HCl was 0.6 mole.

8 g Sodium exchanged clay was dispersed in 2 litre distilled water. The slurry was stirred for 8 h by mechanical stirrer. The Titanium polycation pillaring agent was added to the slurry slowly with vigorous stirring at 90°C. The solution contained 10 m mole titanium/g clay. The resulting solution was further stirred for 4 h. The solution was kept 18 h at room temperature. The mass was filtered under suction and thoroughly washed with distilled water to make Cl⁻ ion free. The product is oven dried at 110±10°C for 6 h. Finally it was calcined at 350°C for 12 h in a muffle furnace.

Preparation of Vanadyl oxalate: 45 g vanadium pentoxide in water(1:5) was heated over steam bath and 122 g oxalic acid crystals slowly added pinch wise with stirring till the colour of the slurry changes from yellow to violet blue. It was further heated over steam bath for 15 minutes. Thus the solution ofvanadyl oxalate was prepared.

Solution A was prepared by diluting 18 ml vanadyl oxalate solution containing 9.35 g vanadyl oxalate(equivalent to 5.49 g vanadium pentoxide) with 75 g distilled water and 0.95 g H₃PO₄ (85%) was added and mixed. Solution B was prepared by dissolving 17.6 g ammonium hepta molybdate in 65 g distilled water. Both solutions were heated at 70-80 °C, solution A was added to solution B and 10.5 g Ti-pillared clay was added and mixed thoroughly. This mixed mass was then heated over steam bath till the mass reduced to about half. Semi dried mass of the catalyst was then oven dried at 110-120 °C for 15 h and then calcined at 300°C for 3 hours and further at 400-425 °C for 15 h. This was then pelletised (6 mm dia) and sized to -6+14 mesh size.

The reaction was carried out using a down flow, fixed bed, pyrex glass reactor of 20 mm I.D. The reactor was packed with 8.8 ml (13.2 g ) Ti-PILC based catalyst. The reaction mixtures, 3-Picoline and water in the ratio 1:3 (by vol.) was fed at the rate 6.0 ml/h using a syringe pump (sage instrument). Ammonia gas (2.4 l/h) from gas cylinder and air (4.5 l/h) through calibrated flow meter were fed in to the reactor. The reaction was carried out at temperature of 430 ± 10°C and contact time 1.05. The product was cooled using ice cooled water and collected at the bottom followed by two number of ice traps. The product was analysed by gas chromatograph using 2 m length carbowax 20 M column at 160°C temperature and Thermal Conductivity Detector. The yield of 3- cyanopyridine obtained was 93.0 % the conversion 96.1 %.

**The main advantages of the present invention are:**
1. The catalyst is prepared in aqueous medium.
2. The said catalyst is active, stable and low cost material.

## Claims

1. A pillared clay based vanadia catalyst useful for the conversion of 3-picoline to 3-cyanopyridine, said catalyst comprising vanadium, phosphorous and molybdenum on a Ti-pillared clay support.

2. A catalyst as claimed in claim 1 wherein the clay is pillared with polyoxymetal cations of titanium and has titania clusters as the pillars between the layers.

3. A catalyst as claimed in claim 1 wherein the clay is selected from a single smectite clay and mixed layer smectite clays.

4. A catalyst as claimed in claim 3 wherein the single smectite clay comprises montmorillonite clay.

5. A catalyst as claimed in claim 3 wherein the mixed layered smectite clay is selected from rectorite and paragonite.

6. A catalyst as claimed in claim 1 wherein the pillared clay contains one or more rare earth elements selected from cerium, lanthanum and a mixture thereof

7. A catalyst as claimed in claim 1 wherein the ratio of the compounds of vanadium, molybdenum, phosphorous over titanium pillared clay in the catalyst is in a ratio ranging between 1.0: 2.5: 0.5: 20 - 1.0: 30: 1.5: 50.

8. A catalyst as claimed in claim 1 wherein the pillared clay contains a homogeneous distribution of pillars in the interlayered spaces thereof forming an array of rectangular openings or pores of a size in the range of 7 to 20 Angstroms high and between 8 and 20 Angstroms wide, enabling the pillared clay to perform like a two-dimensional crystalline molecular sieve.

9. A process for the preparation of a pillared clay based vandia catalyst comprising first preparing vanadyl oxalate by heating a vanadium rich compound on a water bath followed by addition of oxalic acid till the colour of the mass changes to violet blue indicating the formation of vanadyl oxalate, said vanadyl oxalate being in dilute solution, adding a phosphorous source to the dilute solution of vanadyl oxalate heated over water bath, followed by addition of an aqueous solution of molybdenum source and mixing while adding a Titanium source under stirring, evaporating the solution over water bath till the mass is reduced to about half, further heating the mass till a powder catalyst is obtained.

10. A process as claimed in claim 9 wherein the vanadium rich compound is selected from the group consisting of ammonium metavanadate, vanadyl sulphate and oxides of vanadium.

11. A process as claimed in claim 10 wherein the oxide of vanadium is vanadium pentoxide.

12. A process as claimed in claim 9 wherein the phosphorous source is selected from the group consisting of ortho-phosphoric acid, pyro-phosphoric acid and meta-phosphoric acid.

13. A process as claimed in claim 9 wherein the molybdenum source is ammonium molybdate.

14. A process as claimed in claim 9 wherein the titanium source is titanium-pillared clay.

15. A process as claimed in claim 14 wherein the clay is pillared with polyoxymetal cations of titanium and has titania clusters as the pillars between the layers.

16. A process as claimed in claim 14 wherein the clay is selected from a single smectite clay and mixed layer smectite clays.

17. A process as claimed in claim 16 wherein the single smectite clay comprises montmorillonite clay.

18. A process as claimed in claim 16 wherein the mixed layered smectite clay is selected from rectorite and paragonite.

19. A process as claimed in claim 14 wherein the pillared clay contains one or more rare earth elements selected from cerium, lanthanum and a mixture thereof

20. A process as claimed in claim 9 wherein the ratio of the compounds of vanadium, molybdenum, phosphorous over titanium pillared clay in the catalyst is in a ratio ranging between 1.0: 2.5: 0.5: 20 - 1.0: 30: 1.5: 50.

21. A process as claimed in claim 9 wherein the reaction is carried out at a space velocitie in the range of 1500 to 5500 h⁻¹ and after dilution of the catalyst with an inert medium to a range in the extent of 0.5 to 4 by volume with respect to the volume of catalyst.

22. A process as claimed in claim 9 wherein the reduced mass is heated at a temperature of about 110°C in an air oven for 15 hours and then further heated in a muffle furnace at a temperature of about 300°C for 3 hours and subsequently at a temperature of about 425°C for 15 hours.

23. A process as claimed in claim 9 wherein the catalyst powder obtained is pelletized and sized to -6 to +14 mesh size.

24. A process as claimed in claim 9 wherein the vanadium rich compound comprises vanadium pentoxide, said process comprising heating vanadium pentoxide with water in the range of 1:3 to 1:5 (w/v) followed by addition of oxalic acid in the range of 2.5 to 3.0 parts by weight of vanadium pentoxide till the colour of the mass changes to violet blue; adding phosphorous source to the dilute solution of vanadyl oxalate in the range of 2 to 4 times by volume of vanadyl oxalate over heating bath at a temperature in the range of 70 to 80°C; preparing aqueous solution of molybdenum source in the range of 1:4 to 1:6 (w/v) and heating over a heating bath at a temperature in the range of 70 to 80°C; mixing both the solutions in heating condition over heating bath with appropriate amount of titanium-pillared clay under stirring; evaporating the mixture over a heating bath till the mass reduces to half; heating the mass in an air oven at a temperature in the range of 100 to 120°C for a time period in the range of 10 to 16 hours and in a muffle furnace at a temperature in the range of 250 to 350°C for a time period in the range of 16 to 20 hours and pelletizing the same size range of -6 to +14 BSS.

25. A process for the conversion of 3-picoline into 3-cyanopyridine in a single step, said process comprising reacting 3-picoline, ammonia and air in the presence of a Ti-pillared clay based vanadia catalyst to obtain 3-cyanopyridine, without requiring addition of oxidizing agents.

26. A process as claimed in claim 25 wherein the clay is pillared with polyoxymetal cations of titanium and has titania clusters as the pillars between the layers.

27. A process as claimed in claim 26 wherein the clay is selected from a single smectite clay and mixed layer smectite clays.

28. A process as claimed in claim 27 wherein the single smectite clay comprises montmorillonite clay.

29. A process as claimed in claim 27 wherein the mixed layered smectite clay is selected from rectorite and paragonite.

30. A process as claimed in claim 27 wherein the pillared clay contains one or more rare earth elements selected from cerium, lanthanum and a mixture thereof.

31. A process as claimed in claim 26 wherein the ratio of the compounds of vanadium, molybdenum, phosphorous over titanium pillared clay in the catalyst is in a ratio ranging between 1.0: 2.5: 0.5: 20 - 1.0: 30: 1.5: 50.

32. A process as claimed in claim 26 wherein the pillared clay contains a homogeneous distribution of pillars in the interlayered spaces thereof forming an array of rectangular openings or pores of a size in the range of 7 to 20 Angstroms high and between 8 and 20 Angstroms wide, enabling the pillared clay to perform like a two-dimensional crystalline molecular sieve.

## Patentansprüche

1. Vanadiumoxidkatalysator auf der Basis von verbrücktem Ton, welcher für die Umwandlung von 3-Picolin in 3-Cyanopyridin geeignet ist, wobei der Katalysator Vanadium, Phosphor und Molybdän auf einem Träger aus Ti-verbrücktem Ton umfasst.

2. Katalysator nach Anspruch 1, wobei der Ton mit Polyoxymetallkationen des Titans verbrückt ist und Titandioxid-Cluster als Brücken zwischen den Schichten aufweist.

3. Katalysator nach Anspruch 1, wobei der Ton aus einem einfachen Smektitton und Smektittonen mit gemischten Schichten ausgewählt ist.

4. Katalysator nach Anspruch 3, wobei der einfache Smektitton Montmorillonitton umfasst.

5. Katalysator nach Anspruch 3, wobei der Smektitton mit gemischten Schichten aus Rectorit und Paragonit ausgewählt ist.

6. Katalysator nach Anspruch 1, wobei der verbrückte Ton ein oder mehrere Seltenerdelemente enthält, die aus Cer, Lanthan und einem Gemisch aus diesen ausgewählt sind.

7. Katalysator nach Anspruch 1, wobei das Verhältnis der Vanadium-, Molybdän-, Phosphor-Verbindungen gegenüber dem titanverbrückten Ton in dem Katalysator im Bereich von 1,0 : 2,5 : 0,5 : 20 bis 1,0 : 30 : 1,5 : 50 liegt.

8. Katalysator nach Anspruch 1, wobei der verbrückte Ton eine homogene Verteilung der Brücken in den Schichtzwischenräumen enthält, wodurch ein Feld von rechtwinkligen Öffnungen oder Poren einer Höhe im Bereich von 7 bis 20 Ångström und einer Breite im Bereich von 8 bis 20 Ångström gebildet wird, wodurch ermöglicht wird, dass der verbrückte Ton wie ein zweidimensionales kristallines Molekularsieb wirkt.

9. Verfahren zur Herstellung eines Vanadiumoxidkatalysators auf der Basis von verbrücktem Ton, wobei das Verfahren das Folgende umfasst: Herstellen von Vanadyloxalat durch Erwärmen einer vanadiumreichen Verbindung in einem Wasserbad, gefolgt von der Zugabe von Oxalsäure, bis sich die Farbe der Masse in ein Blauviolett verändert, wodurch die Bildung von Vanadyloxalat angezeigt wird, wobei das Vanadyloxalat in verdünnter Lösung vorliegt, Hinzugeben einer Phosphorquelle zu der überm Wasserbad erwärmten verdünnten Vanadyloxalat-Lösung, gefolgt von der Zugabe einer wässrigen Lösung einer Molybdänquelle und Mischen, während unter Rühren eine Titanquelle hinzu gegeben wird, Verdampfen der Lösung überm Wasserbad, bis die Masse auf ungefähr die Hälfte verringert ist, und weiteres Erwärmen der Masse, bis ein pulverförmiger Katalysator erhalten ist.

10. Verfahren nach Anspruch 9, wobei die vanadiumreiche Verbindung aus der Gruppe ausgewählt ist, die aus Ammoniummetavanadat, Vanadylsulfat und Oxiden des Vanadiums besteht.

11. Verfahren nach Anspruch 10, wobei es sich bei dem Oxid des Vanadiums um Vanadiumpentoxid handelt.

12. Verfahren nach Anspruch 9, wobei die Phosphorquelle aus der Gruppe ausgewählt ist, die aus Orthophosphorsäure, Pyrophosphorsäure und Metaphosphorsäure und besteht.

13. Verfahren nach Anspruch 9, wobei es sich bei der Molybdänquelle um Ammoniummolybdat handelt.

14. Verfahren nach Anspruch 9, wobei es sich bei der Titanquelle um titanverbrückten Ton handelt.

15. Verfahren nach Anspruch 14, wobei der Ton mit Polyoxymetallkationen des Titans verbrückt ist und Titandioxid-Cluster als Brücken zwischen den Schichten aufweist.

16. Verfahren nach Anspruch 14, wobei der Ton aus einem einfachen Smektitton und Smektittonen mit gemischten Schichten ausgewählt ist.

17. Verfahren nach Anspruch 16, wobei der einfache Smektitton Montmorillonitton umfasst.

18. Verfahren nach Anspruch 16, wobei der Smektitton mit gemischten Schichten aus Rectorit und Paragonit ausgewählt ist.

19. Verfahren nach Anspruch 14, wobei der verbrückte Ton ein oder mehrere Seltenerdelemente enthält, die aus Cer, Lanthan und einem Gemisch aus diesen ausgewählt sind.

20. Verfahren nach Anspruch 9, wobei das Verhältnis der Vanadium-, Molybdän-, Phosphor-Verbindungen gegenüber dem titanverbrückten Ton in dem Katalysator im Bereich von 1,0 : 2,5 : 0,5 : 20 bis 1,0 : 30 : 1,5 : 50 liegt.

21. Verfahren nach Anspruch 9, wobei die Reaktion mit einer Raumgeschwindigkeit im Bereich von 1.500 bis 5.500 h⁻¹ und nach der Verdünnung des Katalysators mit einem inerten Medium in einer Menge im Bereich des 0,5 bis 4-fachen in Bezug auf das Volumen des Katalysators durchgeführt wird.

22. Verfahren nach Anspruch 9, wobei die verringerte Masse 15 Stunden lang in einem Trockenschrank auf eine Temperatur von ungefähr 110 °C erwärmt wird und dann in einem Muffelofen 3 Stunden lang weiter auf eine Temperatur von ungefähr 300 °C und anschließend 15 Stunden lang auf eine Temperatur von ungefähr 425 °C erwärmt wird.

23. Verfahren nach Anspruch 9, wobei das erhaltene Katalysatorpulver pelletiert und auf eine Korngröße von -6 bis +14 gebracht wird.

24. Verfahren nach Anspruch 9, wobei die vanadiumreiche Verbindung Vanadiumpentoxid umfasst, wobei das Verfahren das Erwärmen von Vanadiumpentoxid mit Wasser im Bereich von 1:3 bis 1:5 (w/v), gefolgt von der Zugabe von Oxalsäure im Bereich von 2,5 bis 3,0 Gewichtsteilen in Bezug auf das Vanadiumpentoxid, bis sich die Farbe der Masse in ein Blauviolett verändert; das Hinzugeben einer Phosphorquelle zu der verdünnten Vanadyloxalat-Lösung im Bereich des 2- bis 4-fachen des Volumens des Vanadyloxalats überm Wärmebad einer Temperatur im Bereich von 70 bis 80 °C; das Herstellen einer wässrigen Lösung einer Molybdänquelle im Bereich von 1:4 bis 1:6 (w/v) und das Erwärmen über einem Wärmebad einer Temperatur im Bereich von 70 bis 80 °C; das Vermischen beider Lösungen unter Erwärmungsbedingungen überm Wärmebad mit einer geeigneten Menge an Titan-verbrücktem Ton unter Rühren; das Verdampfen des Gemisches über einem Wärmebad, bis die Masse auf die Hälfte verringert ist; das Erwärmen der Masse in einem Trockenschrank auf eine Temperatur im Bereich von 100 bis 120 °C für eine Zeitdauer im Bereich von 10 bis 16 Stunden und in einem Muffelofen bei einer Temperatur im Bereich von 250 bis 350 °C für eine Zeitdauer im Bereich von 10 bis 16 Stunden und das Pelletieren auf den Größenbereich von -6 bis +14 BSS umfasst.

25. Verfahren zur Umwandlung von 3-Picolin in 3-Cyanopyridin in einem einzigen Schritt, wobei das Verfahren die Reaktion von 3-Picolin, Ammoniak und Luft in Gegenwart eines Vanadiumoxidkatalysators auf der Basis von Ti-verbrücktem Ton umfasst, um 3-Cyanopyridin zu erhalten, ohne dass die Zugabe von Oxidationsmitteln erforderlich ist.

26. Verfahren nach Anspruch 25, wobei der Ton mit Polyoxymetallkationen des Titans verbrückt ist und Titandioxid-Cluster als Brücken zwischen den Schichten aufweist.

27. Verfahren nach Anspruch 26, wobei der Ton aus einem einfachen Smektitton und Smektittonen mit gemischten Schichten ausgewählt ist.

28. Verfahren nach Anspruch 27, wobei der einfache Smektitton Montmorillonitton umfasst.

29. Verfahren nach Anspruch 27, wobei der Smektitton mit gemischten Schichten aus Rectorit und Paragonit ausgewählt ist.

30. Verfahren nach Anspruch 27, wobei der verbrückte Ton ein oder mehrere Seltenerdelemente enthält, die aus Cer, Lanthan und einem Gemisch aus diesen ausgewählt sind.

31. Verfahren nach Anspruch 26, wobei das Verhältnis der Vanadium-, Molybdän-, Phosphor-Verbindungen gegenüber dem titanverbrückten Ton in dem Katalysator im Bereich von 1,0 : 2,5 : 0,5 : 20 bis 1,0 : 30 : 1,5 : 50 liegt.

32. Verfahren nach Anspruch 26, wobei der verbrückte Ton eine homogene Verteilung der Brücken in den Schichtzwischenräumen enthält, wodurch ein Feld von rechtwinkligen Öffnungen oder Poren einer Höhe im Bereich von 7 bis 20 Ångström und einer Breite im Bereich von 8 bis 20 Ångström gebildet wird, wodurch ermöglicht wird, dass der verbrückte Ton wie ein zweidimensionales kristallines Molekularsieb wirkt.

## Revendications

1. Catalyseur de vanadium à base d'argile à pilier utile pour la conversion de la 3-picoline en 3-cyanopyridine, ledit catalyseur comprenant du vanadium, du phosphore et du molybdène sur un support d'argile à pilier de Ti.

2. Catalyseur selon la revendication 1, dans lequel l'argile a pour piliers des cations polyoxymétaliques de titane et a des amas de dioxyde de titane comme piliers entre les couches.

3. Catalyseur selon la revendication 1, dans lequel l'argile est choisi parmi une argile de smectite simple et des argiles de smectite à couches mixtes.

4. Catalyseur selon la revendication 3, dans lequel l'argile de smectite simple comprend l'argile de montmorillonite.

5. Catalyseur selon la revendication 3, dans lequel l'argile de smectite à couches mixtes est choisie parmi la rectorite et la paragonite.

6. Catalyseur selon la revendication 1, dans lequel l'argile à pilier contient un ou plusieurs éléments de terres rares choisis parmi le cérium, le lanthane et un mélange de ceux-ci.

7. Catalyseur selon la revendication 1, dans lequel le rapport des composés de vanadium, de molybdène, de phosphore sur l'argile à pilier de titane dans le catalyseur est un rapport entre 1,0 : 2,5 : 0,5 : 20 - 1,0 : 30 : 1,5 : 50.

8. Catalyseur selon la revendication 1, dans lequel l'argile à pilier contient une distribution homogène des piliers dans les espaces inter-couches de celle-ci, formant une série d'ouvertures ou pores rectangulaires d'une taille allant de 7 à 20 Ǻngström de hauteur et entre 8 et 20 Ǻngström de largeur, permettant à l'argile à pilier de faire effet de tamis moléculaire cristallin bidimensionnel.

9. Procédé de préparation d'une argile à pilier à base d'un catalyseur de vanadium comprenant tout d'abord la préparation d'oxalate de vanadyle en chauffant un composé riche en vanadium au bain marie puis en ajoutant de l'acide oxalique jusqu'à ce que la couleur de la masse passe à un bleu violet, indiquant la formation d'oxalate de vanadyle, ledit oxalate de vanadyle étant en solution diluée, en ajoutant une source de phosphore à la solution diluée d'oxalate de vanadyle chauffée au bain marie puis en ajoutant une solution aqueuse de source de molybdène et en mélangeant tout en ajoutant une source de titane en agitant, en évaporant la solution sur le bain marie jusqu'à ce que la masse soit réduite à peu près de moitié, en chauffant encore 1a masse jusqu'à obtenir un catalyseur en poudre.

10. Procédé selon la revendication 9, dans lequel le composé riche en vanadium est choisi dans le groupe comprenant le métavanadate d'ammonium, le sulfate de vanadyle et des oxydes de vanadium.

11. Procédé selon la revendication 10, dans lequel l'oxyde de vanadium est le pentoxyde de vanadium.

12. Procédé selon la revendication 9, dans lequel la source de phosphore est choisie dans le groupe comprenant l'acide ortho-phosphorique, l'acide pyrophosphorique et l'acide méta-phosphorique.

13. Procédé selon la revendication 9, dans lequel la source de molybdène est le molybdate d'ammonium.

14. Procédé selon la revendication 9, dans lequel la source de titane est une argile à pilier de titane.

15. Procédé selon la revendication 14, dans lequel l'argile a comme pilier des cations polyoxymétaliques de titane et a des amas d'oxyde de titane comme pilier entre les couches.

16. Procédé selon la revendication 14, dans lequel l'argile est choisi parmi une argile de smectite simple et des argiles de smectite à couches mixtes.

17. Procédé selon la revendication 16, dans lequel l'argile de smectite simple comprend l'argile de montmorillonite.

18. Procédé selon la revendication 16, dans lequel l'argile de smectite à couches mixtes est choisie parmi la rectorite et la paragonite.

19. Procédé selon la revendication 14, dans lequel l'argile à pilier contient un ou plusieurs éléments de terres rares choisis parmi le cérium, le lanthane et un mélange de ceux-ci.

20. Procédé selon la revendication 9, dans lequel le rapport des composés de vanadium, de molybdène, de phosphore sur l'argile à pilier de titane dans le catalyseur est un rapport entre 1,0 : 2,5 : 0,5 : 20 - 1,0 : 30 : 1,5 : 50.

21. Procédé selon la revendication 9, dans lequel la réaction est réalisé à une vélocité spatiale de l'ordre de 1500 à 5500 h⁻¹ et après dilution du catalyseur avec un milieu inerte, jusqu'à un ordre de grandeur de 0,5 à 4 en volume par rapport au volume de catalyseur.

22. Procédé selon la revendication 9, dans lequel la masse réduite est chauffée à une température d'environ 110°C dans une étuve à air pendant 15 heures puis chauffée à nouveau dans un four à moufle à une température d'environ 300°C pendant 3 heures et ensuite, à une température d'environ 425°C pendant 15 heures.

23. Procédé selon la revendication 9, dans lequel la poudre de catalyseur est agglomérée et dimensionnée à une taille de -6 à + 14 mesh.

24. Procédé selon la revendication 9, dans lequel le composé riche en vanadium comprend du pentoxyde de vanadium, ledit procédé comprenant le chauffage du pentoxyde de vanadium avec de l'eau à hauteur de 1 : 3 à 1 : 5 (p/v) puis l'addition d'acide oxalique à hauteur de 2,5 à 3,0 parties en poids de pentoxyde de vanadium, jusqu'à ce que la couleur de la masse passe au bleu violet ; l'addition d'une source de phosphore à la solution diluée d'oxalate de vanadyle à hauteur de 2 à 4 fois en volume l'oxalate de vanadyle en chauffant au bain marie à une température de l'ordre de 70 à 80°C ; la préparation d'une solution aqueuse de source de molybdène à hauteur de 1 : 4 à 1 : 6 (p/v) et le chauffage au bain marie à une température de l'ordre de 70 à 80°C ; le mélange des deux solutions dans les conditions de chauffage au bain marie avec une quantité appropriée d'argile à pilier de titane en agitant ; l'évaporation du mélange au bain marie jusqu'à ce que la masse soit réduite de moitié, le chauffage de la masse dans une étuve à air à une température de l'ordre de 100 à 120°C pendant une durée de l'ordre de 10 à 16 heures et dans un four à moufle à une température de l'ordre de 250 à 350°C pendant une durée de l'ordre de 16 à 20 heures et l'agglomération dans le même ordre de grandeur de -6 à +14 BSS.

25. Procédé de conversion d'une 3-picoline en 3-cyanopyridine en une étape unique, ledit procédé comprenant la réaction de la 3-picoline, d'ammoniaque et d'air en présence d'une argile à pilier de Ti à base d'un catalyseur de vanadium pour obtenir la 3-cyanopyridine sans avoir besoin d'ajouter des agents oxydants.

26. Procédé selon la revendication 25, dans lequel l'argile a pour piliers des cations polyoxymétalliques de titane et a des amas d'oxyde de titane comme piliers entre les couches.

27. Procédé selon la revendication 26, dans lequel l'argile est choisie parmi une argile de smectite simple et des argiles de smectite à couches mixtes.

28. Procédé selon la revendication 27, dans lequel l'argile de smectite simple comprend l'argile de montmorillonite.

29. Procédé selon la revendication 27, dans lequel l'argile de smectite à couches mixtes est choisie parmi la rectorite et la paragonite.

30. Procédé selon la revendication 27, dans lequel l'argile à pilier contient un ou plusieurs éléments de terres rares choisis parmi le cérium, le lanthane et un mélange de ceux-ci.

31. Procédé selon la revendication 26, dans lequel le rapport des composés de vanadium, de molybdène, de phosphore sur l'argile à pilier de titane dans le catalyseur est un rapport entre 1,0 : 2,5 : 0,5 : 20 - 1,0 : 30 : 1,5 : 50.

32. Procédé selon la revendication 26, dans lequel l'argile à pilier contient une distribution homogène des piliers dans les espaces inter-couches de celle-ci, formant une série d'ouvertures ou pores rectangulaires d'une taille allant de 7 à 20 Ǻngström en hauteur et entre 8 et 20 Ǻngström de largeur, permettant à l'argile à pilier de faire effet de tamis moléculaire cristallin bidimensionnel.
